# EUROPEAN PATENT APPLICATION

(11) **EP 4 684 828 A1**
(43) Date of publication of application: **28.01.2026**
(21) Application number: 24190309.5
(22) Date of filing: 23.07.2024
(51) Int. Cl.: A61N 5/06

(54) **LIGHT-BASED SKIN THERAPY APPARATUS, AND METHOD**

(71) Applicant: Koninklijke Philips N.V., 5656 AG Eindhoven (NL)
(72) Inventor: TANG, Lijuan, 5656 AG Eindhoven (NL); LIN, Fu-Lung, 5656 AG Eindhoven (NL)
(74) Representative: Philips Intellectual Property & Standards

(57) **Abstract**

There is provided a light-based skin therapy apparatus comprising: a therapy light source adapted to provide therapy light to a skin; a thermoelectric device; an air pump; a first heat exchange surface; a second heat exchange surface; a third heat exchange surface; and an air conduit to provide a first air path and a second air path. The first air path is from the first heat exchange surface to the third heat exchange surface. The second air path is along the second heat exchange surface. The first heat exchange surface is adapted to receive heat from air flowing along the first air path. The thermoelectric device is adapted to transfer heat from the first heat exchange surface to the second heat exchange surface. The third heat exchange surface is adapted to transfer heat from the therapy light source to air flowing along the first air path. The second heat exchange surface is adapted to transfer heat to air flowing along the second air path.

## Description

### FIELD OF THE INVENTION

The invention relates to a light-based skin therapy apparatus, in particular to an intense pulsed light (IPL) apparatus. The invention further relates a method for cooling a light-based skin therapy apparatus.

### BACKGROUND OF THE INVENTION

Intense Pulsed Light (IPL) technology is a popular solution for many applications, such as photo-epilation, lesion treatment, photo-rejuvenation, in-home personal care, professional personal care, and medical settings. For photo-epilation in a home environment, IPL photo-epilation apparatus applies broad-spectrum light to the surface of the skin, targeting the melanin in the hair and hair follicles. Hairs and follicles that are in their anagen phase of the growth cycle absorb the light's energy and go into a resting phase.

An IPL apparatus has a therapy light source that generates therapy light. The therapy light interacts with the skin to create the desired effect in or on the skin, such as the photo-epilation, lesion treatment, and photo-rejuvenation. As a side effect of generating the therapy light, the therapy light source generates a substantial amount of heat. Without cooling of the therapy light source, the temperature of the IPL apparatus would increase to an unacceptably high temperature that could burn the user or damage components of the IPL apparatus.

To remove the heat from therapy light source, a known IPL apparatus draws ambient air into the IPL apparatus. The air flows along the therapy light source. The therapy light source transfers heat to the flow of air, which causes the temperature of the therapy light source to decrease. The heated air flows through an outlet and leaves the IPL apparatus.

### SUMMARY OF THE INVENTION

However, a large flow of air is needed to sufficiently cool the therapy light source. To generate the large flow of air, a large and expensive fan is needed. Also, the air conduit that guides the flow of air through the IPL apparatus has to have a large cross-section to accommodate the large flow of air. As a result, the known IPL apparatus is bulky and expensive.

It is an objective of the invention to provide improved cooling of the therapy light source.

According to a first aspect, there is provided a light-based skin therapy apparatus comprising a therapy light source adapted to provide therapy light to a skin; a thermoelectric device; an air pump; a first heat exchange surface; a second heat exchange surface; a third heat exchange surface; and an air conduit adapted to provide a first air path and a second air path. The first air path is from the first heat exchange surface to the third heat exchange surface. The second air path is along the second heat exchange surface. The air pump is adapted to cause air to flow along the first air path and along the second air path. The first heat exchange surface is adapted to receive heat from air flowing along the first air path. The thermoelectric device is adapted to transfer heat from the first heat exchange surface to the second heat exchange surface. The third heat exchange surface is adapted to transfer heat from the therapy light source to air flowing along the first air path. The second heat exchange surface is adapted to transfer heat to air flowing along the second air path.

The first heat exchange surface is adapted to receive heat from the air flowing along the first air path. Because the first heat exchange surface receives the heat from the air, the temperature of the flow of air along the first air path is reduced. As the first heat exchange surface is upstream of the third heat exchange surface, the flow of air with the reduced temperature is then in contact with the third heat exchange surface to receive heat from the therapy light source. The thermoelectric device is able to transfer heat from the first heat exchange surface to the second heat exchange surface more efficiently, because the heat from the second heat exchange surface is transferred to air flowing along the second air path. Because of the transfer of heat between the second heat exchange surface and the air flowing along the second air path, the thermoelectric device is able to transfer more heat from the first heat exchange surface to the second heat exchange surface. By transferring more heat from the first heat exchange surface to the second heat exchange surface, improved cooling of the air flowing along the first heat exchange surface is achieved. The improved cooling of the air flowing along the first heat exchange surface improves cooling of the therapy light source. The improved cooling allows more heat to be removed from the therapy light source or allows using a smaller flow of air to provide the desired cooling.

The light-based skin therapy apparatus is, for example, adapted to remove hair or to photo-rejuvenate the skin, or to treat veins, or to treat acne. For example, the light-based skin therapy apparatus is an Intense Pulsed Light (IPL) apparatus.

The therapy light source is, for example, adapted to provide light pulses. For example, the therapy light source is adapted to generate light at a high intensity for a short duration, such as for less than 1 second or less than 0.5 second. The intensity of the light pulse is high enough to perform an operation on the skin. Such operation is, for example, hair removal or photo-rejuvenation or vein treatment or acne treatment. For example, the therapy light source comprises one or more optical filters. For example, the therapy light source comprises an optical filter to prevent light with a potentially damaging wavelength, such as UV light, from being transmitted to the skin. For example, the therapy light source comprises a Xenon flash lamp, or a laser, or multiple lasers, or a Light Emitting Diode (LED) or multiple LEDs. For example, the therapy light source is adapted to provide therapy light with wavelengths in the range of 530-1200 nanometers.

The thermoelectric device is adapted to transfer heat from the first heat exchange surface to the second heat exchange surface. By transferring heat from the first heat exchange surface to the second heat exchange surface, the first heat exchange surface becomes cooler compared to if no heat is transferred, whereas the second heat exchange surface becomes hotter compared to if no heat is transferred. The first heat exchange surface may be referred to as the "cool side", whereas the second heat exchange surface may be referred to as the "hot side". The thermoelectric device is adapted to transfer heat in response to receiving electric power. For example, the thermoelectric device is a Peltier element. For example, the thermoelectric device is a thermoelectric cooler. For example, the first heat exchange surface is a surface of the thermoelectric device, whereas the second heat exchange surface is another surface of the thermoelectric device. For example, a surface of the thermoelectric device is in thermal contact with the first heat exchange surface, whereas another surface of the thermoelectric device is in thermal contact with the second heat exchange surface.

The air pump is adapted to cause air to flow along the first air path and along the second air path. For example, the air pump comprises an impeller or a fan to generate a flow of air along the first air path and along the second air path. For example, the air pump has an electric motor to drive the impeller or the fan. For example, the air pump is a centrifugal air pump, or a reciprocating air pump, or an axial air pump.

The first heat exchange surface is, for example, a surface of the thermoelectric device. The first heat exchange surface is, for example, a surface of the air conduit. The surface of the air conduit is in thermal contact with the thermoelectric device. For example, the first heat exchange surface is a smooth surface to allow for air to flow along the first air path with a minimal pressure loss. For example, the first heat exchange surface has protrusions, such as fins or pins, to increase the surface area of the first heat exchange in comparison to the surface area without the protrusions. The increased surface area allows for an improved heat transfer between the air flowing along the first air path and the first heat exchange surface.

The second heat exchange surface is, for example, another surface of the thermoelectric device. The second heat exchange surface is, for example, another surface of the air conduit. The other surface of the air conduit is in thermal contact with the thermoelectric device. For example, the second heat exchange surface is a smooth surface to allow for air to flow along the second air path with a minimal pressure loss. For example, the second heat exchange surface has protrusions, such as fins or pins, to increase the surface area of the second heat exchange in comparison to the surface area without the protrusions. The increased surface area allows for an improved heat transfer between the air flowing along the second air path and the second heat exchange surface.

The third heat exchange surface is, for example, a surface of the therapy light source. The third heat exchange surface is, for example, yet another surface of the air conduit. The yet another surface of the air conduit is in thermal contact with the therapy light source. For example, the third heat exchange surface is a smooth surface to allow for air to flow along the first air path with a minimal pressure loss. For example, the third heat exchange surface has protrusions, such as fins or pins, to increase the surface area of the third heat exchange in comparison to the surface area without the protrusions. The increased surface area allows for an improved heat transfer between the air flowing along the first air path and the third heat exchange surface.

The air conduit is adapted to provide a first air path and a second air path. For example, the air conduit forms a single channel from an air inlet to an air outlet. For example, the air conduit forms multiple parallel channels. For example, the air conduit comprises a hose and/or a hollow section of a casing of the light-based skin therapy apparatus. For example, part of the air conduit is formed by one or more components of the light-based skin therapy apparatus. The one or more components form an internal space in the light-based skin therapy through which air is able to flow along the first air path and/or the second air path. For example, the air conduit comprises one or more of the first heat exchange surface, the second heat exchange surface, and the third heat exchange surface. For example, the air conduit has an opening to receive the thermoelectric device. For example, when in the opening, the thermoelectric device brings the first heat exchange surface in contact with air flowing along the first air path. For example, when in the opening, the thermoelectric device brings the second heat exchange surface in contact with air flowing along the second air path. For example, the air conduit has two openings, wherein one of the openings receives the first heat exchange surface of the thermoelectric device, and wherein the other of the openings receives the second heat exchange surface of the thermoelectric device. For example, the air conduit has an opening to receive the therapy light source. For example, when in the opening, the therapy light source brings the third heat exchange surface in contact with air flowing along the first air path.

In an embodiment, the second air path is from the third heat exchange surface to the second heat exchange surface.

According to the embodiment, the air pump causes air to flow along the first air path from the first heat exchange surface to the third heat exchange surface. The heat from the therapy light source causes the air in contact with the third heat exchange surface to heat up. The heated air then further flows along the second air path from the third heat exchange surface to the second heat exchange surface. Because the thermoelectric element transfers heat from the first heat exchange surface to the second heat exchange surface, the second heat exchange surface has a higher temperature than the heated air along the second air path. Therefore, the second heat exchange surface is able to transfer heat to the heated air along the second air path. As a result, the thermoelectric device is able to provide improved cooling of the air flowing along the first air path.

In an embodiment, the light-based skin therapy apparatus comprises a skin contact surface adapted to contact the skin during use of the light-based skin therapy apparatus. The thermoelectric device is adapted to transfer heat from the skin contact surface to the second heat exchange surface.

Use of the light-based skin therapy apparatus may cause discomfort, pain or damage to the skin, especially when the therapy light is emitted with a high fluence. The skin contact surface is a surface of the light-based skin therapy apparatus that is in physical contact with the skin during use, i.e., the skin contact surface touches the skin during use. By using the thermoelectric device to transfer heat from the skin contact surface to the second heat exchange surface, the skin contact surface is cooled. By cooling the skin contact device, discomfort, pain, and/or skin damage caused by the therapy light is reduced. Because the air flowing along the second air path removes heat from the second heat exchange surface, the thermoelectric device is able to transfer more heat from the skin contact surface, which improves cooling of the skin contact surface. For example, the thermoelectric device transfers heat from the skin contact surface to have a temperature of the skin contact surface of about 20°C.

In an embodiment, the light-based skin therapy apparatus comprises a heat conductive element arranged between the skin contact surface and the second heat exchange surface. The thermoelectric device is adapted to transfer heat from the skin contact surface via the heat conductive element to the second heat exchange surface.

According to the embodiment, the thermoelectric device is able to transfer heat from the skin contact surface via the heat conductive element to the second heat exchange surface. The head portion of the light-based skin therapy apparatus has several components, such as the therapy light source, a window through which the therapy light exits the light-based skin therapy apparatus, and the skin contact surface. As a result, it is challenging to create a design to fit all these components. By using the heat conductive element, only the skin contact surface is in the head portion, whereas the second heat exchange surface, and optionally the thermoelectric device, is not in the head portion, but somewhere else in the light-based skin therapy apparatus. As a result, the light-based skin therapy apparatus can be made less bulky and therefore more user friendly. The heat conductive element has good properties in view of thermal conductivity. For example, the heat conductive element has a higher thermal conductivity than other parts of the light-based skin therapy apparatus, such as the casing the light-based skin therapy apparatus. For example, the heat conductive element comprises a material with a thermal conductivity of at least 50 W/mK or more, such as at least 90 W/mK, or at least 100 W/mK, or at least 200 W/mK. For example, the heat conductive element comprises aluminum or copper or a ceramic such as aluminum oxide, or aluminum nitride, or boron nitride, or beryllium oxide, or silicon carbide. For example, the heat conductive element comprises a heat pipe. A heat pipe comprises a fluid that is adapted to go through a phase transition to transfer heat.

In an embodiment, the heat conductive element has a first end and a second end. The heat conductive element extends from the first end to the second end away from the skin contact surface. The first heat exchange surface is arranged at the first end. The second heat exchange surface is arranged at the second end. The thermoelectric device is arranged at one of the first end and the second end.

According to the embodiment, the heat conductive element extends away from the skin contact surface. As a result, heat is transferred from the skin contact surface to the second end away from the skin contact surface. At the second end, the heat is transferred to the second heat exchange surface. As the second heat exchange surface is at the second end, the second heat exchange surface is away from the skin contact surface. By transferring the heat away from the skin contact surface, it is prevented that the skin at the skin contact surface is exposed to heated air flowing from the second heat exchange surface.

In an embodiment, the thermoelectric device is adapted to cool air flowing along the first air path via the first heat exchange surface to below an ambient temperature.

According to the embodiment, the thermoelectric device removes heat from the air flowing along the first air path, which causes the temperature of the air flowing along the first heat exchange surface to become lower than the ambient temperature. For example, the ambient temperature is room temperature. For example, the ambient temperature is in the range of 15-35°C, or within a range of 18-25°C. For example, the first heat exchange surface has a temperature in the range of 10-20°C, such as 15-18°C. For example, the air flowing along the first air path via the first heat exchange surface has an decreased temperature in the range of 10-20°C, such as 15-18°C.

In an embodiment, the thermoelectric device is adapted to heat air flowing along the second air path via the second heat exchange surface.

According to this embodiment, the thermoelectric device transfers heat to the air flowing along the second air path, which causes the temperature of the air flowing along the second heat exchange surface to become higher than the ambient temperature. For example, the second heat exchange surface has a temperature in the range of 40-60°C, such as 45-50°C. For example, the air flowing along the second air path via the second heat exchange surface has an increased temperature of about 30-40°C, such as about 35°C.

In an embodiment, the thermoelectric device comprises a Peltier element.

In an embodiment, the first heat exchange surface and/or the second heat exchange surface and/or the third heat exchange comprises fins.

According to the embodiment, the fins increase the surface area in comparison to the surface area without the fins. The increased surface area allows for an improved heat transfer between the air flowing the first heat exchange surface, the second heat exchange surface, and/or the third heat exchange surface.

In an embodiment, the air conduit comprises an air inlet and an air outlet. The first air path is from the air inlet via the first heat exchange surface to the third heat exchange surface. The second air path is from the third heat exchange surface via the second heat exchange surface to the air outlet.

According to the embodiment, the air pump causes air to flow through the air conduit from the air inlet via the first heat exchange surface to the third heat exchange surface, and from the third heat exchange surface via the second heat exchange surface to the air outlet. So air flowing along the first air path continues to flow along the second air path. The air is cooled at the first heat exchange surface, which allows the cooled air to receive more heat from the third heat exchange surface. This improves cooling of the therapy light source. The air heated by the third heat exchange surface is further heated by the second heat exchange surface. This improves the amount of heat the thermoelectric device is able to transfer away from the first heat exchange surface. This improves cooling of the air flowing along the first air path. For example, the temperature of the third heat exchange surface is lower than the temperature of the second heat exchange surface when the light-based skin therapy apparatus is in use. For example, the third heat exchange surface is adapted to raise the temperature of air flowing along the third heat exchange surface to a temperature lower than the temperature of the second heat exchange surface. For example, the thermoelectric device is adapted to raise the temperature of the second heat exchange surface to above the temperature of the third heat exchange surface.

In an embodiment, the light-based skin therapy apparatus comprises an air path divider. The air conduit is adapted to provide a main air path. The air path divider is adapted to divide air flowing from the main air path between the first air path and the second air path. The air pump is adapted to cause air to flow via the main air path to the first air path and the second air path.

According to the embodiment, the air path divider creates two parallel air paths, i.e., the first air path and the second air path. The first air path is along the first heat exchange surface and the third heat exchange surface. The air flowing along the first air path provides cooling to the therapy light source. The second air path is along the second heat exchange surface. The air flowing along the second air path provides cooling of the second heat exchange surface. This allows the thermoelectric device to transfer more heat from the first heat exchange surface to the second heat exchange surface. As a result, the thermoelectric device is able to cool the air along the first air path better.

In an embodiment, the light-based skin therapy apparatus comprises a first outlet and a second outlet. The first air path extends between the air path divider and the first outlet. The second air path extends between the air path divider and the second outlet.

According to the embodiment, air flowing along the first air path exists the light-based skin therapy apparatus via the first outlet. Air flowing along the second air path exists the light-based skin therapy apparatus via the second outlet.

In an embodiment, the air pump is arranged along the main air path.

According to the embodiment, the air pump causes air to flow along the main air path towards the air path divider. The air path divider is adapted to divide the flowing air into two parallel flows. One flow of air continues via the first air path. The other flow of air continues via the second air path. For example, the air path divider is adapted to generate a first flow of air along the first air path at a first flow rate, and to generate a second flow of air along the second air path at a second flow rate, wherein the first flow rate and the second flow rate are different from each other. For example, the first flow rate is higher than the second flow rate. In case the first flow rate is higher than the second flow rate, more air flows along the first air path than along the second air path per unit of time. For example, the first flow rate is lower than the second flow rate. In case the first flow rate is lower than the second flow rate, less air flows along the first air path than along the second air path per unit of time. By using different flow rates along the first flow path and the second flow path, the heat transfer from the second heat exchange surface and the third heat exchange surface may be optimized. For example, the air path divider comprises a cross-section for the first air path and a cross-section for the second air path different from the cross-section for the first air path. A flow rate is defined as the volume of air per unit of time, for example, liters per second or cubic meters per minute.

In an embodiment, the air path divider is adapted to adjust a ratio between a first flow rate and a second flow rate. The first flow rate is of air flowing along the first air path. The second flow rate is of air flowing along the second air path.

According to the embodiment, the air path divider is adapted to change the ratio between first flow rate and the second flow rate. A desired second flow rate, for example, 0.06 m³/min, provides sufficient cooling of the thermoelectric device that allows the thermoelectric device to operate at a desired heat transferring capacity. Providing further cooling of the thermoelectric device may not be desired, as this would improve the heat transferring capacity only marginally or not at all, while the air pump requires additional energy to generate the additional flow rate. A desired first flow rate depends on the use of the therapy light source. For example, when the light-based skin therapy device is in use only for a short while, or when in use with at a low intensity level of the therapy light, the temperature of the therapy light source may still be relatively low. Therefore, the desired first flow rate is low, for example, 0.03 m³/min. In this situation, the air pump provides a main flow rate along the main air path of 0.06+0.03=0.09 m³/min. The air path divider divides the main flow rate in the desired first flow rate along the first air path and the desired second flow rate along the second air path. The ratio between the first flow rate and the second flow rate is 0.03/0.06 = 0.5. However, when the light-based skin therapy device is in use for a long while, or when in use with at a high intensity level of the therapy light, the temperature of the therapy light source may become high. Therefore, the desired first flow rate becomes high, for example 0.09 m³/min, to provide sufficient cooling of the therapy light source. The desired second flow rate is still 0.06 m³/min to operate the thermoelectric device at the desired heat transferring capacity. The air pump provides a main flow rate along the main air path of 0.06+0.09=0.15 m³/min. The air path divider divides the main flow rate in the desired first flow rate along the first air path and the desired second flow rate along the second air path. The ratio between the first flow rate and the second flow rate is 0.09/0.06 = 1.5. This way, the air flow generated by the air pump is used in an efficient way. By adjusting the ratio, it is prevented that an excessive amount of air flows along the second air path, where that excessive amount of air does not help to improve the heat transferring capacity of the thermoelectric device. In comparison, in case the air path divider would not be adapted to adjust the ratio, the ratio would be 0.5 to have a sufficiently large second flow rate when the desired first flow rate is 0.03 m³/min. For the desired first flow rate of 0.09 m³/min, the ration would still be the same 0.5 instead of 1.5. For the desired first flow rate of 0.09 m³/min, the second flow rate would be 0.09/0.5=0.18 m³/min. This value is higher than the desired second flow rate of 0.06 m³/min, and thus 0.12 m³/min of air would be wasted. As a result of adjusting the ratio, a smaller air pump is sufficient to cool the light-based skin therapy apparatus and/or less power is needed to cool the light-based skin therapy apparatus. For example, the air divider comprises an air valve to control a cross-section of the first air path or the second air path. By changing the position of the air valve, the cross-section is changed. As a result, the flow of air through the cross-section is changed. This way, the ratio is adjusted. For example, the air valve is arranged in the first air path. For example, the air valve is arranged in the second air path. For example, the air divider comprises two air valves, one air valve arranged in the first air path, and another air valve arranged in the second air path. For example, the air valve comprises a solenoid or a step motor. For example, the air valve has a limited number of positions, such as two positions or three positions or more than three positions. For example, the air valve is adapted to set the position anywhere in a range of positions.

In an embodiment, the air pump is adapted to provide air along the main path at at least a high flow rate and a low flow rate. The high flow rate is higher than the low flow rate. The air path divider is adapted to adjust the ratio to have a larger ratio for the high flow rate than for the low flow rate.

According to this embodiment, the air path divider is adapted to adjust the ratio to have a larger ratio for the high flow rate than for the low flow rate. When providing the high flow rate, a larger portion of the main flow rate is to be provided to the first air path compared to when providing the lower flow rate. To use a similar example as above, the low flow rate is 0.09 m³/min, the desired first flow rate is 0.03 m³/min, and the desired second flow rate is 0.06 m³/min. The ratio in this case is 0.5. For example, the high flow rate is 0.15 m³/min, the desired first flow rate is 0.09 m³/min, and the desired second flow rate is still 0.06 m³/min. This results in a ratio of 0.09/0.06 = 1.5.

In an embodiment, the air path divider is adapted to adjust the ratio to maintain the second flow rate at a same level for the high flow rate and the low flow rate.

According to the embodiment, the ratio is adjusted to maintain the same flow rate along the second air path independent of the main flow rate generated by the air pump. The level of the second flow rate is set, for example, to optimize the heat transfer capacity of the thermoelectric element.

In an embodiment, the therapy light source is adapted to provide therapy light at at least a high intensity level and a low intensity level. The high intensity level is higher than the low intensity level. The air pump is adapted to provide the high flow rate in case the therapy light source provides the therapy light at the high intensity level. The air pump is adapted to provide the low flow rate in case the therapy light source provides the therapy light at the low intensity level.

According to this embodiment, the therapy light source is adapted to provide the therapy light at multiple intensity levels. For example, depending on a characteristic of the skin that is to be treated, the intensity level may be different. For example, in case the therapy light is for hair removal, a low intensity level is sufficient to remove dark hairs in a light skin, whereas a high intensity level is needed to remove light hairs in a light skin, or to remove dark hairs from a dark skin. For example, a low intensity level is used to treat a sensitive skin area, such as the face, whereas a high intensity level is used to treat a less sensitive skin area, such as arms and legs. The therapy light source generates more heat at the high intensity level than at the low intensity level. Therefore, the air pump provides the high flow rate at the high intensity level and the low flow rate at the low intensity level.

In an embodiment, the light-based skin therapy apparatus comprises a temperature sensor adapted to generate a temperature signal representative of a temperature of the therapy light source. The air pump is adapted to provide the high flow rate when the temperature signal exceeds a temperature threshold. The air pump is adapted to provide the low flow rate when the temperature signal does not exceed the temperature threshold.

According to the embodiment, the air pump provides the low flow rate when the temperature of the therapy light source is not above the temperature threshold. When the therapy light source is at this low temperature, the low flow rate provides sufficient cooling. However, the temperature of the therapy light source may rise due to prolonged use of the light-based skin therapy apparatus and/or applying the high intensity level. When the temperature of the therapy light source exceeds the temperature threshold, the air pump provides the high flow rate to provide sufficient cooling.

In a second aspect of the invention, there is provided a method for cooling a light-based skin therapy apparatus. The light-based skin therapy apparatus comprises a therapy light source adapted to provide therapy light to a skin. The light-based skin therapy apparatus comprises a first heat exchange surface, a second heat exchange surface, and a third heat exchange surface. The method comprises flowing air along a first air path from the first heat exchange surface to the third heat exchange surface. The method comprises flowing air along a second air path along the second heat exchange surface. The method comprises transferring heat from the air flowing along the first air path to the first heat exchange surface. The method comprises transferring heat from the first heat exchange surface to the second heat exchange surface. The method comprises transferring heat from the therapy light source via the third heat exchange surface to the air flowing along the first air path. The method comprises transferring heat from the second heat exchange surface to the air flowing along the second air path.

In an embodiment, the light based therapy apparatus comprises a skin contact surface adapted to contact the skin during use of the light based therapy apparatus. The method comprises transferring heat from the skin contact surface to the second heat exchange surface.

In an embodiment, the method comprises dividing a main air path in the first air path and the second air path. The method comprises flowing air via the main air path to the first air path and the second air path.

In an embodiment, the method comprises adjusting a ratio between a first flow rate and a second flow rate. The first flow rate is of air flowing along the first air path. The second flow rate is of air flowing along the second air path.

In an embodiment, the method comprises providing air along the main path at a low flow rate. The method comprises providing air along the main path at a high flow rate. The high flow rate is higher than the low flow rate. The method comprises adjusting the ratio to have a larger ratio for the high flow rate than for the low flow rate.

In an embodiment, the method comprises adjusting the ratio to maintain the second flow rate at a same level for the high flow rate and the low flow rate.

In an embodiment, the method comprises providing the therapy light at a low intensity level. The method comprises providing the therapy light at a high intensity level. The high intensity level is higher than the low intensity level. The method comprises providing the high flow rate while providing the therapy light at the high intensity level. The method comprises providing the low flow rate while providing the therapy light at the low intensity level. For example, the fluence of the therapy light at the high intensity level is higher than at the low intensity level. For example, the number of pulses of the therapy light at the high intensity level is higher than at the low intensity level.

In an embodiment, the method comprises generating a temperature signal representative of a temperature of the therapy light source. The method comprises providing the high flow rate when the temperature signal exceeds a temperature threshold. The method comprises providing the low flow rate when the temperature signal does not exceed the temperature threshold.

### BRIEF DESCRIPTION OF THE DRAWINGS

Exemplary embodiments will now be described, by way of example only, with reference to the following Figures, in which:
FIG. 1 depicts a light-based skin therapy apparatus according to a first embodiment of the invention;
FIG. 2 depicts a cross-section of the light-based skin therapy apparatus according to the first embodiment;
FIG. 3 depicts a cross-section of the light-based skin therapy apparatus according to a second embodiment of the invention;
FIGs. 4A and 4B depict a detail of the second embodiment;
FIGs. 5A and 5B depict a detail of a third embodiment of the invention;
FIG. 6 depicts a fourth embodiment of the invention;
FIG. 7 depicts a cross-section of the fourth embodiment;
FIG. 8 depicts a method for cooling a light-based skin therapy apparatus according to a fifth embodiment.

### DETAILED DESCRIPTION OF THE EMBODIMENTS

It should be understood that the detailed description and specific examples, while indicating exemplary embodiments of the apparatus and methods, are intended for purposes of illustration only and are not intended to limit the scope of the invention. These and other features, aspects, and advantages of the apparatus and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

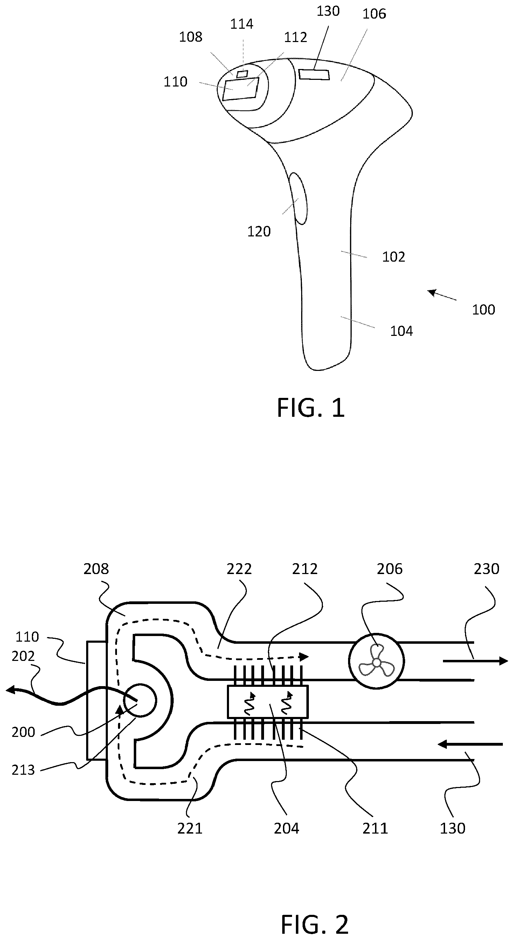

FIG. 1 depicts a light-based skin therapy apparatus according to a first embodiment of the invention. The light-based skin therapy apparatus 100 is an Intense Pulsed Light (IPL) apparatus. The light-based skin therapy apparatus 100 is adapted for use on the skin of a subject, e.g. a person or an animal. The light-based skin therapy apparatus 100 comprises a housing 102 that has a handle portion 104 and a head portion 106. The handle portion 104 is shaped to enable the user to hold the light-based skin therapy apparatus 100. The head portion 106 has a head end 108 that is to be placed into contact with the subject in order for the light-based skin therapy apparatus 100 to perform a treatment operation on the skin of the subject.

The light-based skin therapy apparatus 100 comprises therapy light source 200 adapted to provide therapy light to the skin. The therapy light source 200 is arranged inside the head portion 106. The light-based skin therapy apparatus 100 performs the treatment operation by providing pulses of the therapy light 202 to the skin via the aperture 110. The aperture 110 is arranged in or on the housing 102 so that the aperture 110 can be placed adjacent to or on (i.e. in contact with) the skin of the subject. A window 112 is arranged in the aperture 110. The intensity of the light pulses from the therapy light source 200 are high enough to perform the treatment operation on the skin or body part adjacent to the aperture 110.

The light-based skin therapy apparatus 100 comprises a user control 120 adapted to be operated by the user to activate the light-based skin therapy apparatus 100. When the user activates the light-based skin therapy apparatus 100 via the user control 120, the light-based skin therapy apparatus 100 is able to perform the treatment operation. The user control 120 may be in the form of a switch, a button, a touch pad, etc.

The light-based skin therapy apparatus 100 comprises a sensor window 114 for emitting sensor light to the skin for measuring a characteristic of the skin. The light-based skin therapy apparatus 100 comprises an air inlet 130.

FIG. 2 depicts a cross-section of the light-based skin therapy apparatus 100 according to the first embodiment.

The light-based skin therapy apparatus 100 comprises a therapy light source 200 adapted to provide therapy light 202 to a skin. The light-based skin therapy apparatus 100 comprises a thermoelectric device 204, an air pump 206, a first heat exchange surface 211, a second heat exchange surface 212, a third heat exchange surface 213, and an air conduit 208. The air conduit 208 is adapted to provide a first air path 221 and a second air path 222. The first air path 221 is from the first heat exchange surface 211 to the third heat exchange surface 213. The second air path 222 is along the second heat exchange surface 212. The air pump 206 is adapted to cause air to flow along the first air path 221 and along the second air path 222. The first heat exchange surface 211 is adapted to receive heat from air flowing along the first air path 221. The thermoelectric device 204 is adapted to transfer heat from the first heat exchange surface 211 to the second heat exchange surface 212. The transfer of heat is schematically depicted with the arrows. The third heat exchange surface 213 is adapted to transfer heat from the therapy light source 200 to air flowing along the first air path 221. The second heat exchange surface 212 is adapted to transfer heat to air flowing along the second air path 222. The second air path 222 is from the third heat exchange surface 213 to the second heat exchange surface 212.

The thermoelectric device 204 is adapted to cool air flowing along the first air path 221 via the first heat exchange surface 211 to below an ambient temperature. The thermoelectric device 204 is adapted to heat air flowing along the second air path 222 via the second heat exchange surface 212. The thermoelectric device 204 comprises a Peltier element. The first heat exchange surface 211 and the second heat exchange surface 212 comprise fins. Optionally, the third heat exchange surface 213 comprises fins.

The air conduit 208 comprises the air inlet 130 and the air outlet 230. The first air path 221 is from the air inlet 130 via the first heat exchange surface 211 to the third heat exchange surface 213. The second air path 222 is from the third heat exchange surface 213 via the second heat exchange surface 212 to the air outlet 230.

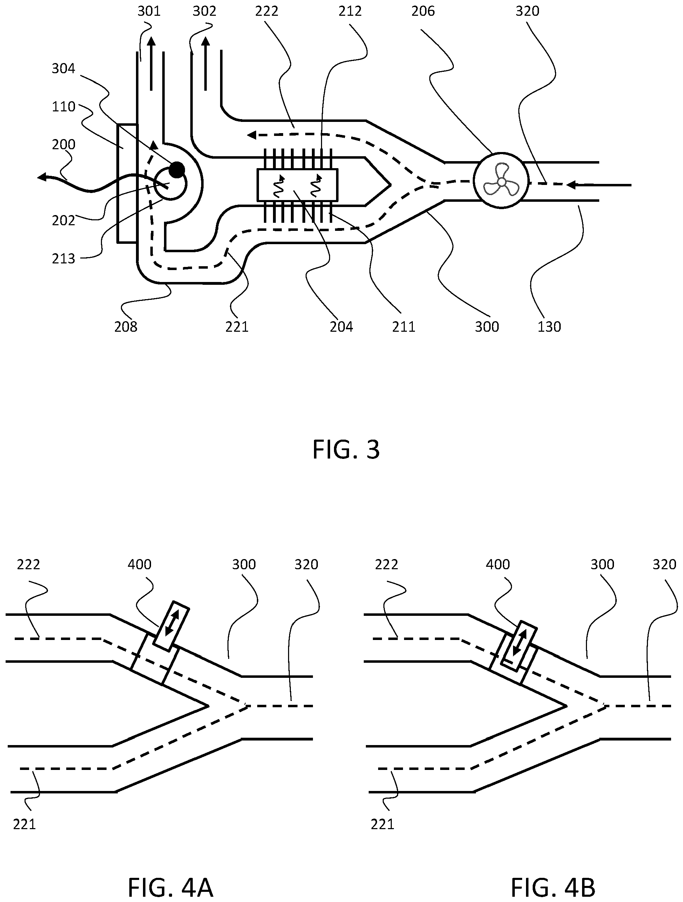

FIG. 3 depicts a cross-section of the light-based skin therapy apparatus 100 according to a second embodiment. The second embodiment has, for example, the same features as the first embodiment, except for the following.

The light-based skin therapy apparatus 100 comprises an air path divider 300. The air conduit 208 is adapted to provide a main air path 320. The air path divider 300 is adapted to divide air flowing from the main air path 320 between the first air path 221 and the second air path 222. The air pump 206 is adapted to cause air to flow via the main air path 320 to the first air path 221 and the second air path 222.

The light-based skin therapy apparatus 100 comprises a first air outlet 301 and a second air outlet 302. The first air path 221 extends between the air path divider 300 and the first air outlet 301. The second air path 222 extends between the air path divider 300 and the second air outlet 302. The air pump 206 is arranged along the main air path 320.

FIGs. 4A and 4B depict a detail of the second embodiment. The detail shows a close-up of the air path divider 300. The air path divider 300 is adapted to adjust a ratio between a first flow rate and a second flow rate. The first flow rate is of air flowing along the first air path 221. The second flow rate is of air flowing along the second air path 222.

The air path divider 300 comprises an air valve 400 arranged along the second air path 222. The air valve 400 has multiple positions. In FIG. 4A, the air valve 400 is in an open position. In the open position, the air valve 400 provides a large cross-section for air flowing along the second air path 222. In FIG. 4B, the air valve 400 is in a partly closed position. In the partly closed position, the air valve 400 provides a cross-section for air flowing along the second air path 222 that is smaller than the cross-section corresponding to the open position.

The air pump 206 provides air along the main air path 320. The air path divider 300 divides the air from the main air path 320 between the first air path 221 and the second air path 222. If the air valve 400 is in the open position, a large portion of the air from the main air path 320 flows along the second air path 222. As a result, the ratio between the first flow rate and the second flow rate is low. If the air valve 400 is in the partly closed position, a smaller portion of the air from the main air path 320 flows along the second air path 222. As a result, the ratio between the first flow rate and the second flow rate is higher.

The air pump 206 is adapted to provide air along the main path at at least a high flow rate and a low flow rate. The high flow rate is higher than the low flow rate. The air path divider 300 is adapted to adjust the ratio to have a larger ratio for the high flow rate than for the low flow rate.

The air path divider 300 is adapted to adjust the ratio to maintain the second flow rate at a same level for the high flow rate and the low flow rate.

The therapy light source 200 is adapted to provide therapy light 202 at at least a high intensity level and a low intensity level. The high intensity level is higher than the low intensity level. The air pump 206 is adapted to provide the high flow rate in case the therapy light source 200 provides the therapy light 202 at the high intensity level. The air pump 206 is adapted to provide the low flow rate in case the therapy light source 200 provides the therapy light 202 at the low intensity level.

The following table shows an example of the first flow rate and the second flow rate for different intensity levels of the therapy light source 200.

**Table 1: example flow rates and intensity levels**

| Intensity level | Total flow rate [%] | First flow rate [%] | Second flow rate [%] | Ratio [-] |
|---|---|---|---|---|
| 1 | 100 | 80 | 20 | 4:1 |
| 2 | 150 | 130 | 20 | 6.5:1 |
| 3 | 200 | 180 | 20 | 9:1 |

The table shows intensity levels 1-3. The lowest intensity level is 1, whereas the highest intensity level is 3. The total flow rate is the flow rate along the main air path 320. The total flow rate is the sum of the first flow rate and the second flow rate. The ratio is the ratio between the first flow rate and the second flow rate. Further, the total flow rate at intensity level 1 is set at 100%. The other values of the total flow rate, the first flow rate, and the second flow rate are relative to that value to provide a clear comparison.

At intensity level 1, the first flow rate is 80% of the total flow rate, whereas the second flow rate is 20% of the total flow rate. As a result, the ratio is 4:1.

At intensity level 2, more cooling of the therapy light source 200 is needed. Therefore, the total flow rate increased to 150% compared to intensity level 1. The second flow rate is still 20% of the total flow rate at intensity level 1, as this is sufficient for proper cooling of the thermoelectric device 204. This leaves a first flow rate of 130% for cooling the therapy light source 200. So the increase of the total flow rate of 50% is directed completely along first air path 221. The ratio is now 6.5:1.

At intensity level 3, even more cooling of the therapy light source 200 is needed. Therefore, the total flow rate increased to 200% compared to intensity level 1. The second flow rate is still 20% of the total flow rate at intensity level 1, as this is sufficient for proper cooling of the thermoelectric device 204. This leaves a first flow rate of 180% for cooling the therapy light source 200. So the additional increase of the total flow rate of 50% is directed completely along first air path 221. The ratio is now 9: 1.

The following is to compare to a situation without adjusting the ratio. The ratio is set to 4:1 to have sufficient cooling of the thermoelectric element at the intensity level 1. To cool the therapy light source 200 at the intensity level 3, the first flow rate of 180% is needed. With the ratio of 4:1, this would give a second flow rate of 180/4=45%, which is more than double the desired second flow rate of 20%.

For example, the total flow rate at intensity level 1 of 100% is in the range of 1-10 cubic feet per minute, or in the range of 1-7 cubic feet per minute or in the range of 3-5 cubic feet per minute.

For example, the total flow rate at intensity level 1 of 100% is in the range of 0.03-0.5 m³/min, or in the range of 0.05-0.3 m³/min, or in the range of 0.07-0.15 m³/min.

Further referring to FIG. 3, the light-based skin therapy apparatus 100 optionally comprises a temperature sensor 304. The temperature sensor 304 is adapted to generate a temperature signal representative of a temperature of the therapy light source 200. The air pump 206 is adapted to provide the high flow rate when the temperature signal exceeds a temperature threshold. The air pump 206 is adapted to provide the low flow rate when the temperature signal does not exceed the temperature threshold.

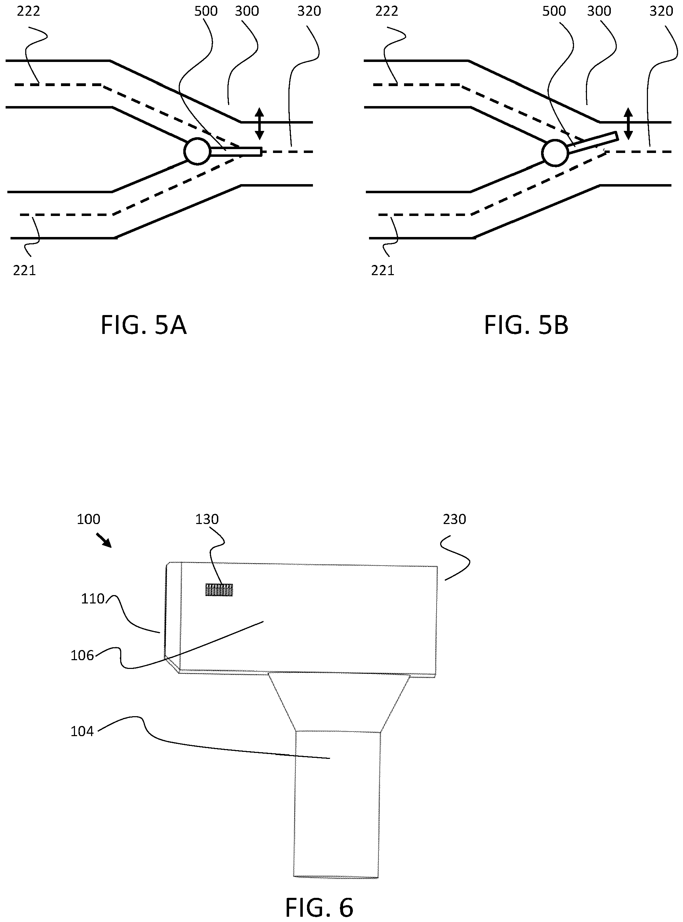

FIGs 5A and 5B depict a detail of a third embodiment. The third embodiment has, for example, the same elements as the second embodiment, except for the following.

The detail shows a close-up of the air path divider 300. The air path divider 300 is adapted to adjust the ratio between a first flow rate and a second flow rate. The first flow rate is of air flowing along the first air path 221. The second flow rate is of air flowing along the second air path 222.

Instead of the air valve 400, the air path divider 300 comprises a rotatable body 500. The rotatable body 500 is arranged in the air path divider 300 to rotate from a first position to a second position. In the first position, as shown in FIG. 5A, the rotatable body 500 does not restrict air flowing from the main air path 320 to the first air path 221, and from the main air path 320 to the second air path 222. In the second position, as shown in FIG. 5B, the rotatable body 500 restricts air flowing from the main air path 320 to the second air path 222, but does not restrict air flowing from the main air path 320 to the first air path 221. As a result, the ratio between the first flow rate and the second flow rate is different in FIG. 5A than in FIG. 5B.

Optionally, the rotatable body 500 is rotatable from the first position to a third position. In the third position, the rotatable body 500 restricts air flowing from the main air path 320 to the first air path 221, but does not restrict air flowing from the main air path 320 to the second air path 222. As a result, the ratio between the first flow rate and the second flow rate is different than in FIG. 5A and FIG. 5B.

FIG. 6 depicts a fourth embodiment of the invention. The fourth embodiment has, for example, the same elements as the first embodiment, as the second embodiment, or as the third embodiment, except for the following.

FIG. 6 depicts a light-based skin therapy apparatus 100 according to the fourth embodiment of the invention. The light-based skin therapy apparatus 100 has the handle portion 104 and the head portion 106. The handle portion 104 is shaped to enable the user to hold the light-based skin therapy apparatus 100.

The light-based skin therapy apparatus 100 comprises therapy light source 200 adapted to provide therapy light 202 to the skin. The therapy light source 200 is arranged inside the head portion 106. The light-based skin therapy apparatus 100 performs the treatment operation by providing pulses of the therapy light 202 to the skin via the aperture 110. The light-based skin therapy apparatus 100 comprises the air inlet 130 and the air outlet 230.

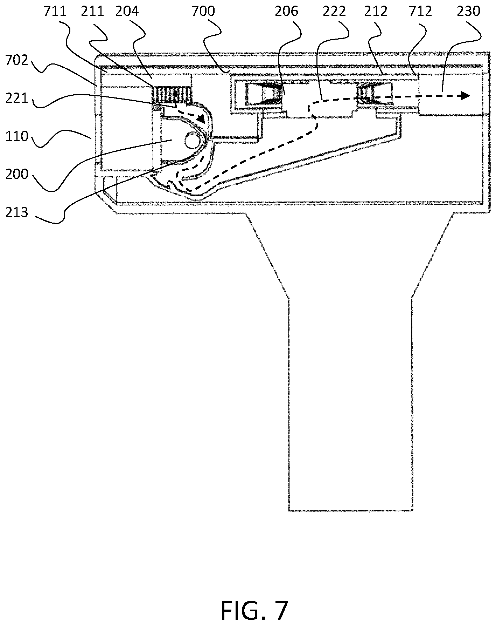

FIG. 7 depicts a cross-section of the fourth embodiment. The first air path 221 is from the air inlet 130 via the first heat exchange surface 211 to the third heat exchange surface 213. The second air path 222 is from the third heat exchange surface 213 along the second heat exchange surface 212 to the outlet. The air pump 206 is adapted to cause air to flow along the first air path 221 and along the second air path 222. The first heat exchange surface 211 is adapted to receive heat from air flowing along the first air path 221. The third heat exchange surface 213 is adapted to transfer heat from the therapy light source 200 to air flowing along the first air path 221. The second heat exchange surface 212 is adapted to transfer heat to air flowing along the second air path 222.

The thermoelectric device 204 is adapted to transfer heat from the first heat exchange surface 211 to the second heat exchange surface 212.

The light-based skin therapy apparatus 100 comprises a skin contact surface 702 adapted to contact the skin during use of the light-based skin therapy apparatus 100. The thermoelectric device 204 is adapted to transfer heat from the skin contact surface 702 to the second heat exchange surface 212. In this embodiment, the skin contact surface 702 is arranged adjacent to and in thermal contact with the thermoelectric device 204.

The light-based skin therapy apparatus 100 comprises a heat conductive element 700 arranged between the skin contact surface 702 and the second heat exchange surface 212. The thermoelectric device 204 is adapted to transfer heat from the skin contact surface 702 via the heat conductive element 700 to the second heat exchange surface 212. The second heat exchange surface 212 is a surface of the heat conductive element 700 that is in contact with air flowing along the second air path 222.

The heat conductive element 700 has a first end 711 and a second end 712. The heat conductive element 700 extends from the first end 711 to the second end 712 away from the skin contact surface 702. The first heat exchange surface 211 is arranged at the first end 711. The second heat exchange surface 212 is arranged at the second end 712. The thermoelectric device 204 is arranged at one of the first end 711 and the second end 712, in this case at the first end 711.

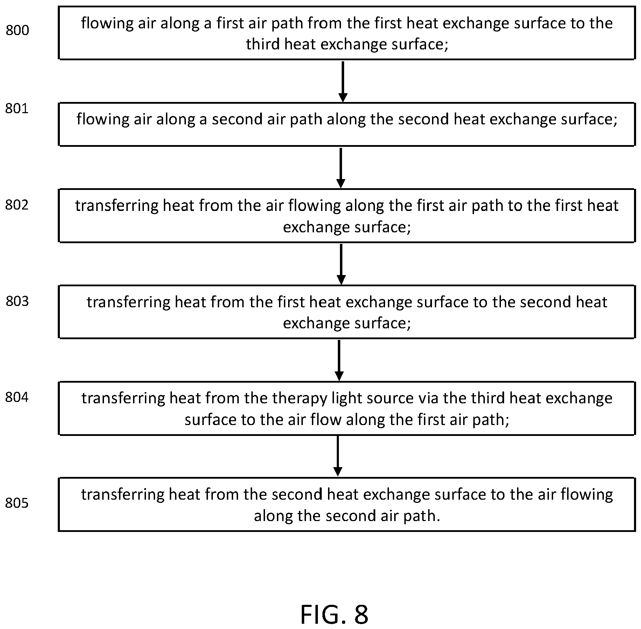

FIG. 8 depicts a method for cooling a light-based skin therapy apparatus 100 according to a fifth embodiment.

The method is for cooling the light-based skin therapy apparatus 100. The light-based skin therapy apparatus 100 comprises a therapy light source 200 adapted to provide therapy light 202 to the skin. The light-based skin therapy apparatus 100 comprises the first heat exchange surface 211, the second heat exchange surface 212, and the third heat exchange surface 213.

The method comprises, at 800, flowing air along a first air path 221 from the first heat exchange surface 211 to the third heat exchange surface 213. The method comprises, at 801, flowing air along a second air path 222 along the second heat exchange surface 212. The method comprises, at 802, transferring heat from the air flowing along the first air path 221 to the first heat exchange surface 211. The method comprises, at 803, transferring heat from the first heat exchange surface 211 to the second heat exchange surface 212. The method comprises, at 804, transferring heat from the therapy light source 200 via the third heat exchange surface 213 to the air flowing along the first air path 221. The method comprises, at 805, transferring heat from the second heat exchange surface 212 to the air flowing along the second air path 222.

Optionally, the light based therapy apparatus comprises the skin contact surface 702 adapted to contact the skin during use of the light based therapy apparatus. The method comprises transferring heat from the skin contact surface 702 to the second heat exchange surface 212.

Optionally, the method comprises dividing the main air path 320 in the first air path 221 and the second air path 222, and flowing air via the main air path 320 to the first air path 221 and the second air path 222.

Optionally, the method comprises adjusting the ratio between a first flow rate and a second flow rate. The first flow rate is of air flowing along the first air path 221. The second flow rate is of air flowing along the second air path 222.

Optionally, the method comprises providing air along the main path at a low flow rate, and providing air along the main path at a high flow rate. The high flow rate is higher than the low flow rate. The method comprises adjusting the ratio to have a larger ratio for the high flow rate than for the low flow rate.

Optionally, the method comprises adjusting the ratio to maintain the second flow rate at a same level for the high flow rate and the low flow rate.

Optionally, the method comprises providing the therapy light 202 at a low intensity level, and providing the therapy light 202 at a high intensity level. The high intensity level is higher than the low intensity level. The method comprises providing the high flow rate while providing the therapy light 202 at the high intensity level. The method comprises providing the low flow rate while providing the therapy light 202 at the low intensity level.

Optionally, the method comprises generating a temperature signal representative of a temperature of the therapy light source 200. The method comprises providing the high flow rate when the temperature signal exceeds a temperature threshold. The method comprises providing the low flow rate when the temperature signal does not exceed the temperature threshold.

The light-based skin therapy apparatus 100 may comprise a processing unit configured to control one or more components of the light-based skin therapy apparatus 100, such as the therapy light source 200, the air pump 206, the thermoelectric device 204, the valve, and/or the rotatable body 500. The processing unit may be embodied as a digital and/or analog processing system.

In various implementations, the processing unit may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems. The processing unit may be configured to execute the computer program, causing the light-based skin therapy apparatus 100 to perform at least part of the method according to the fifth embodiment.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A light-based skin therapy apparatus (100), comprising:
a therapy light source (200) adapted to provide therapy light (202) to a skin;
a thermoelectric device (204);
an air pump (206);
a first heat exchange surface (211);
a second heat exchange surface (212);
a third heat exchange surface (213); and
an air conduit (208) adapted to provide a first air path (221) and a second air path (222),
wherein the first air path (221) is from the first heat exchange surface (211) to the third heat exchange surface (213),
wherein the second air path (222) is along the second heat exchange surface (212),
wherein the air pump (206) is adapted to cause air to flow along the first air path (221) and along the second air path (222),
wherein the first heat exchange surface (211) is adapted to receive heat from air flowing along the first air path (221),
wherein the thermoelectric device (204) is adapted to transfer heat from the first heat exchange surface (211) to the second heat exchange surface (212),
wherein the third heat exchange surface (213) is adapted to transfer heat from the therapy light source (200) to air flowing along the first air path (221),
wherein the second heat exchange surface (212) is adapted to transfer heat to air flowing along the second air path (222).

2. The light-based skin therapy apparatus (100) according to claim 1, wherein the second air path (222) is from the third heat exchange surface (213) to the second heat exchange surface (212).

3. The light-based skin therapy apparatus (100) according to any one of the preceding claims, comprising a skin contact surface (702) adapted to contact the skin during use of the light-based skin therapy apparatus (100),
wherein the thermoelectric device (204) is adapted to transfer heat from the skin contact surface (702) to the second heat exchange surface (212).

4. The light-based skin therapy apparatus (100) according to claim 3, comprising a heat conductive element (700) arranged between the skin contact surface (702) and the second heat exchange surface (212),
wherein the thermoelectric device (204) is adapted to transfer heat from the skin contact surface (702) via the heat conductive element (700) to the second heat exchange surface (212).

5. The light-based skin therapy apparatus (100) according to claim 4, wherein the heat conductive element (700) has a first end (711) and a second end (712),
wherein the heat conductive element (700) extends from the first end (711) to the second end (712) away from the skin contact surface (702),
wherein the first heat exchange surface (211) is arranged at the first end (711),
wherein the second heat exchange surface (212) is arranged at the second end (712), and
wherein the thermoelectric device (204) is arranged at one of the first end (711) and the second end (712).

6. The light-based skin therapy apparatus (100) according to any one of the preceding claims, comprising an air path divider (300),
wherein the air conduit (208) is adapted to provide a main air path (320),
wherein the air path divider (300) is adapted to divide air flowing from the main air path (320) between the first air path (221) and the second air path (222),
wherein the air pump (206) is adapted to cause air to flow via the main air path (320) to the first air path (221) and the second air path (222).

7. The light-based skin therapy apparatus (100) according to claim 6, wherein the air path divider (300) is adapted to adjust a ratio between a first flow rate and a second flow rate,
wherein the first flow rate is of air flowing along the first air path (221),
wherein the second flow rate is of air flowing along the second air path (222).

8. The light-based skin therapy apparatus (100) according to claim 7, wherein the air pump (206) is adapted to provide air along the main path at at least a high flow rate and a low flow rate,
wherein the high flow rate is higher than the low flow rate,
wherein the air path divider (300) is adapted to adjust the ratio to have a larger ratio for the high flow rate than for the low flow rate.

9. The light-based skin therapy apparatus (100) according to claim 8, wherein the air path divider (300) is adapted to adjust the ratio to maintain the second flow rate at a same level for the high flow rate and the low flow rate.

10. The light-based skin therapy apparatus (100) according to claim 8 or 9, wherein the therapy light source (200) is adapted to provide therapy light (202) at at least a high intensity level and a low intensity level,
wherein the high intensity level is higher than the low intensity level,
wherein the air pump (206) is adapted to provide the high flow rate in case the therapy light source (200) provides the therapy light (202) at the high intensity level,
wherein the air pump (206) is adapted to provide the low flow rate in case the therapy light source (200) provides the therapy light (202) at the low intensity level.

11. The light-based skin therapy apparatus (100) according to any one of claims 8-10, comprising a temperature sensor (304) adapted to generate a temperature signal representative of a temperature of the therapy light source (200),
wherein the air pump (206) is adapted to provide the high flow rate when the temperature signal exceeds a temperature threshold,
wherein the air pump (206) is adapted to provide the low flow rate when the temperature signal does not exceed the temperature threshold.

12. A method for cooling a light-based skin therapy apparatus (100), wherein the light-based skin therapy apparatus (100) comprises a therapy light source (200) adapted to provide therapy light (202) to a skin,
wherein the light-based skin therapy apparatus (100) comprises a first heat exchange surface (211), a second heat exchange surface (212), and a third heat exchange surface (213),
wherein the method comprises:
flowing air along a first air path (221) from the first heat exchange surface (211) to the third heat exchange surface (213);
flowing air along a second air path (222) along the second heat exchange surface (212);
transferring heat from the air flowing along the first air path (221) to the first heat exchange surface (211);
transferring heat from the first heat exchange surface (211) to the second heat exchange surface (212);
transferring heat from the therapy light source (200) via the third heat exchange surface (213) to the air flowing along the first air path (221);
transferring heat from the second heat exchange surface (212) to the air flowing along the second air path (222).

13. The method according to claim 12, wherein the light-based therapy apparatus (100) comprises a skin contact surface (702) adapted to contact the skin during use of the light based therapy apparatus, the method comprising:
transferring heat from the skin contact surface (702) to the second heat exchange surface (212).

14. The method according to claim 12 or 13, comprising
dividing a main air path (320) in the first air path (221) and the second air path (222);
flowing air via the main air path (320) to the first air path (221) and the second air path (222).

15. The method according to claim 14, comprising
adjusting a ratio between a first flow rate and a second flow rate,
wherein the first flow rate is of air flowing along the first air path (221),
wherein the second flow rate is of air flowing along the second air path (222).
